Europäisches Patentamt

European Patent Office

Office européen des brevets

(11) Publication number: **0 225 615**
**A2**

(12) **EUROPEAN PATENT APPLICATION**

(21) Application number: **86116914.2**

(22) Date of filing: **05.12.86**

(51) Int. Cl.⁴: **A 61 K 9/22,** A 61 K 9/18, A 61 K 31/485

(30) Priority: **09.12.85 US 806879**

(43) Date of publication of application: **16.06.87** **Bulletin 87/25**

(84) Designated Contracting States: **AT BE CH DE ES FR GB GR IT LI LU NL SE**

(71) Applicant: **CIBA-GEIGY AG, Klybeckstrasse 141, CH-4002 Basel (CH)**

(72) Inventor: **Fischer, Franz Xaver, Dr., Höhenstrasse 27, CH-4125 Riehen (CH)**
Inventor: **Khanna, Satish Chandra, Dr., Rütistrasse 26, CH-4103 Bottmingen (CH)**

(74) Representative: **Zumstein, Fritz, Dr. et al, Bräuhausstrasse 4, D-8000 München 2 (DE)**

(54) **Resinate sustained release dextromethorphan composition.**

(57) The invention relates to a pharmaceutical preparation for controlled, sustained release of dextromethorphan comprising a polystyrene sulfonate resin which has been cross-linked with about 3% to about 10% divinyl benzene, having an average particle size of at least 40 µm and less than 100 µm onto which dextromethorphan has been loaded in a ratio expressed in weight amounts of dextromethorphan-hydrobromide to resin of about 1:6 to about 1:10 and at least one pharmaceutically acceptable adjuvant.

EP 0 225 615 A2

CIBA-GEIGY AG
Basel / Switzerland                                   4-15645/+


Resinate Sustained Release Dextromethorphan Composition


The invention relates to sustained and controlled pharmaceutical
compositions. A number of resinous materials have been known to form
complexes with ionic drug substances which are subsequently released
in the gastrointestinal tract at a controlled rate being lower than
the release rate found for conventional drug delivery systems such
as tablets, syrups, solutions etc. For example, USP 2,990,332
discloses pharmaceutical preparations comprising cationic exchange
resin adsorption compounds wherein the cationic exchange resin is a
sulfonated styrene divinylbenzene copolymerisate loaded with various
basic drugs such as amphetamines, atropine, scopolamin, codein and
others. The release properties of these compositions have been
proved to be unsatisfactory as they provide only a short delay of
drug release in the gastrointestinal part.

It has been proposed in USP 4,221,778 that selective, prolonged and
continuous drug release under physiological conditions such as those
encountered in the gastrointestinal tract are achieved by the
application of diffusion barrier coatings to ion exchange resin drug
complex particles. For example, USP 4,221,778 discloses an
Amberlite® XE-69 (= IRP 69) dextromethorphan composition coated with
an ethylcellulose-vegetable oil layer.

However, the process of coating sulfonated resins has certain
disadvantages as gel-type divinylbenzenesulfonic acid resins swell
when placed in water and shrink in volume when dried. This property
is undesirable for obtaining a diffusion barrier coating in the
drug-resin complex particles. The coating peals from the particles
as soon as they are suspended in the dissolution medium because they are absorbed by the medium and swell rapidly. Attempts to coat the particles in the swollen form fail because the particles do not fluidize satisfactorily in the coating apparatus. A solution to this problem is proposed in J. Pharm. Sc. Vol. 70, Nr. 4, 1981, pages 379-384. The resin-drug complex matrix is filled at least partially with solubilizers such as polyethylene glycol which prevents the ion exchange resin from swelling.

Object of the present invention are sustained and controlled release dextromethorphan compositions having a reasonable delay of drug release in aqueous solution. It is also object of the present invention to prepare sustained and controlled release dextromethorphan compositions by formulation methods that avoid additional process steps such as coating and filling the resin matrix with solubilizing agents such polyethylene glycol.

Surprisingly, these objects have been realized by preparing a dextromethorphan composition that contains a gel-type cationic exchange resin consisting of a sulfonated polystyrene-divinylbenze copolymerisate in an amount that exceeds by weight the amount of dextromethorphan in the composition by at least a factor of five.

Subject matter of the present invention is a pharmaceutical composition suitable for controlled, sustained release of a pharmaceutically acceptable acid addition salt of dextromethorphan. The pharmaceutical composition contains

a) a cationic, gel-type, microporous ion-exchange resin consisting of a sulfonated polystyrene-divinylbenze copolymerisate,
b) a pharmaceutically acceptable acid addition salt of dextromethorphan in a weight ratio of 1:5 to 1:10 expressed for the weight ratio of dextromethorphan-hydrobromide and the Na-form of the resin and
c) at least one pharmaceutically acceptable adjuvant.

The pharmaceutical compositions are useful as antitussive preparations.

The general definitions and terms employed in the description of the present invention preferably have the following meanings:

A cationic, microporous ion-exchange resin consisting of a sulfonated polystyrene-divinylbenzene copolymerisate has the capability of exchanging in aqueous solution mono- or bivalent cations, especially sodium ions, for the quaternary dextromethorphan acid addition salt.

The resin present in the pharmaceutical composition has in dry state an average particle size of about 35-150 μm (100-500 Mesh). Preferred is an average particle size between 40 and 100 μm, more preferably between 40 and 80 μm and most preferably between 50 and 70 μm. It is desirable that about 85 %, preferably 95 %, and most preferably 98 % of the resin particles have sizes within the ranges set forth above. The desired particle sizes are obtained by grinding granulates with conventional grinding methods. The pore diameter is in dry state about 0,5-1,5 nm.

The microporous ion exchange resins employed in the pharmaceutical compositions according to the present invention swell in aqueous solution. The resin has an ion exchange capacity of at least 4,3 meq/g sec and is strongly acidic.

The conditions mentioned above are at present met by resins known as sulfonated styrene-divinylbenzene copolymerisates. Such copolymerisates consist crosslinked styrene polymers obtainable by copolymerization of styrene with divinylbenze acting as polymerizing agent. The crosslinking agent is present to an extent of about 2-12 %, preferably 6-10 % and most preferably 7-9 % based on the weight of the dry resin. The preparation of the styrene-divinylbenzene copolymerisate matrix and the introduction of sulfo groups into the matrix is performed by methods known in the art.

Ion exchange resins consisting of sulfonated styrene-divinylbenzene copolymerisates are well known and documented. A concise review is found in the chapters beginning on page 279, Vol. 13, of Ullmanns Encylopädie der Technischen Chemie, 4th Edition, Verlag Chemie and/or in the chapters beginning on page 678, Vol. 13, Kirk-Othmer, Encyclopedia of Chemical Technology, J. Wiley, and in the numerous references cited in these reviews.

Preferred are ion-exchange resins consisting of sulfonated styrene-divinylbenzene copolymerisates which are commercially available and which have been approved for pharmaceutical purposes for formulation with medicaments, for example resins currently available from Rohm & Haas Co. under the trade names Amberlite® IRP-69 and IRP-69 M.

Dextromethorphan is the d-enantiomer of racemethorphan, see Merck Index Tenth Edition No. 8009 (3-methoxy-17-methylmorphinan) and is classified in the therapeutic category of antitussives. A pharmaceutically acceptable acid addition salt is formed by reaction of the dextromethorphan base with a non-toxic acid, such as aqueous hydrohalic acids, e.g. diluted hydrogen chloride, and especially hydrogen bromide.

The dextromethorphan-resin complex is prepared by admixing the components, especially by pretreating the resin, for example the Amberlite IRP-69 resin, in aqueous solution with an aqueous NaOH-solution in order to convert sulfonic acid groups in the resin to the sodium sulfonate groups. After washing the resin several times with deionized water and drying to constant weight the resin is added to an aqueous solution containing the dextromethorphan-hydrobromide salt. It is also possible to proceed in reverse order and to add the dextromethorphan-hydrobromide salt to an aqueous solution containing the ion exchange resin.

The dextromethorphan-resin complex (components a) and b)) thus
formed can be formulated into liquid or solid compositions which
contain carriers such as solvents, thickeners, preservatives,
coloring agents, flavoring agents, solubilizers, dispersants and
other adjuvants known in the art all of which are pharmaceutically
acceptable.

A preferred liquid formulation contains about 0.3 g to about 1.5 g,
preferably about 1.0 g, of thickener; about 1 g to about 10 g,
preferably about 2.5 g, of 1,2-propylene glycol as a dissolving
agent; about 0.12 g to about 0.19 g, preferably 0.15 g, of at least
one paraben preservative such as methyl paraben; about 0.05 g to
about 0.2 g, preferably about 0.1 g, of sorbic acid; about 30 g to
about 60 g, preferably 40 g, of a sugar alcohol solution; about
0.05 g to about 0.2 g, preferably 0.1 g of an artificial sweetener;
dextromethorphan-resin complex in an amount to yield a desired
strength, preferably about 2.10 g (the amount of a 1:6 complex
needed to deliver equivalent to 60 mg of dextromethorphan-
hydrobromide in a 20 ml adult 12 hours dose); and sufficient water
to bring the volume up to 100 ml.

Suitable thickeners include: tragacanth; bentonite; acacia and lower
alkyl ethers of cellulose (including the hydroxy and carboxy
derivatives of the cellulose ethers); preferably tragacanth.
Exemplaries of the paraben preservatives are $C_1-C_4$ alkyl paraben,
preferably methyl, ethyl, propyl, and butyl paraben. Methyl and
propyl paraben are most preferable. Preferably, both methyl and
propyl paraben are present in the formulation in a ratio of methyl
paraben to propyl paraben of from about 2.5:1 to about 7.5 1,
preferably 4:1. The artificial sweetener is advantageously a form of
saccharin or aspartame, preferably saccharin sodium; however, when
desirable, equivalent sweetening amounts of other known sweetening
agents may be substituted therefor. The sugar alcohol is preferably
sorbitol.

A preferred solid formulation for compression into tablets contains additives such as sugars, e.g. lactose, preferably about 30-40 mg, binders such as cellulose, preferably about 30-40 mg, flow regulators such as silicon dioxide (Aerosil®), preferably about 5-10 mg, disintegrating agents such as cross-linked polyvinyl-poly-pyrrolidon, preferably in an amount of 60-100 mg.

In general solid formulations are tablets, dragées and gelatin capsules containing dextromethorphan admixed with additives such as diluents, e.g. lactose, dextrose, sucrose, mannitol, sorbitol, cellulose, calcium phosphates and/or glycine, b) lubricants, e.g. silica, talcum, stearic acid, especially its magnesium or calcium salts and/or polyethyleneglycol; c) binders, e.g. magnesium aluminium silicate, starch paste, gelatin, tragacanth, methylcellulose, sodium carboxymethylcellulose and/or polyvinylpyrrolidone; if desired, d) disintegrants, e.g. starches, agar, alginic acid or its sodium salt, and/or, e) absorbents, colorants, flavors and sweeteners. Dragée or tablet cores may be provided with suitable coatings, which are more or less resistant to gastric juices. Coating solutions are, for example, concentrated aqueous sugar solutions, which may contain gum arabic, polyvinylpyrrolidone, polyethylene glycol, talcum and/or titanium dioxide. Resistant coatings are obtained with lacquer solutions in organic solvents, such as shellac, acetylcellulose phthalate or hydroxypropylmethylcellulose phthalate in ethanol and the like. Dyestuffs or pigments may be added for identification of brand name and dose. Capsules are either made from hard gelatin, or they are soft, closed capsules made from gelatin and softener, e.g. glycerin or sorbitol. The hard capsules contain either uncompressed powder mixtures, e.g. those mentioned und a) and b), or granulates similar to those used for tablets. In the soft capsules said active ingredients are preferably dissolved or suspended in suitable liquids, such as fatty oils, paraffins or polyethyleneglycols.

The amount of the resinate in the formulation is sufficient to deliver, when administered at one dose every 12 hours, an antitussive effective amount of dextromethorphan over a period of approximately 12 hours to a patient in need of such administration. A typical adult dose of 20 ml will contain approximately 420 mg of resinate to deliver an equivalent of 60 mg of dextromethorphan-hydrobromide when the drug:resin ratio is 1:6 and 2.10 g of resinate are present per 100 ml of formulation. The dosage can be altered analogously to that known for the administration of dextromethorphan which has not been complexed with resin.

In addition to the resinate, further active ingredients may be incorporated into the antitussive formulation as desired. These typically will include antihistamines, decongestants, demulcents and other antitussives. These may be utilized as they are or modified for sustained release as well.

The invention will be further understood by reference to the following examples, which illustrate but do not limit the invention.

Example 1: Preparation of the dextromethorphan-resin complex Sulfonated polystyrene resin having 8 % by weight divinyl benzene cross-links (Amberlite® IRP 69 - Rohm & Haas) is treated with 2N NaOH-solution for 4 hours at 50°C to convert the available sulfonic acid groups to their sodium forms. The supernatant is decanted and the resin is washed 3-4 times with deionized water. The washed resinous gel is filtered and dried to constant weight at 50°C under vacuum.

10 g dextromethorphan hydrobromide and 60 g of the dry resin (calculated on a moisture free basis) are dispersed in deionized water and stirred at 50°C for about 24 hours to obtain essentially total binding of dextromethorphan to the resin. Residual free dextromethorphan is checked for by taking samples at intervals and determining the dextromethorphan content. When the amount of unbound dextromethorphan in solution is neglegible, the resinate is filtered and dried to constant weight at 50°C under vacuum to yield resinate No. 1 (see also Table I, Ex. 3).

Example 2: The process of Example 1 is followed except that the amount of dextromethorphan-hydrobromide is altered so that resinates 2-4 (see below) are prepared.

| Resinate No. | Drug/Resin |
|---|---|
| 2 | 1:5 |
| 3 | 1:4 |
| 4 | 1:3 |

Example 3: The release characteristics of resinates 1-4 are evaluated by the flow-cell method. An amount of each resinate containing the equivalent of 60 mg of active ingredient is placed in cells and an eluent is pumped therethrough at a constant rate of 16 ml/min. The free active ingredient in the eluent is then determined at various intervals. The eluent, for the first 60 minutes is an artificial gastric fluid, (buffered at pH 1.2). Thereafter, the effluent is 0.9 % saline solution.

The results are reported in Table I.

Table I

| Resinate | % release | | | | |
|---|---|---|---|---|---|
| | 1 hr. | 2 hr. | 4 hr. | 5 hr. | 8 hr. |
| 1 | 13 | 38 | 65 | 74 | 88 |
| 2 | 16 | 44 | 71 | 78 | 91 |
| 3 | 24 | 48 | 74 | 81 | 93 |
| 4 | 30 | 60 | 81 | 90 | 97 |

Even when placed 5 hours in solution the resinates of the instant invention still have 10-26 % of their original dose of active agent available for subsequent release. Still further, even after 8 hours, resinates 1 to 4 have 3-12 % of the original dextromethorphan-hydrobromide dose in reserve, making them quite suitable for once in 12 hour dosing.

Example 4: The dextromethorphan-resinate is prepared in accordance with Example 1. The resinate is then screened into three particle size ranges: (1) less than (2) 40 µm, 40 µm-60 µm, and (3) 60 µm-100 µm. Each resinate size group is subjected to flow through cell testing under conditions (rate, eluent, time) analogous to those in Example 3; however, only resinate having a drug:resin ratio of 1:6 is utilized. The results demonstrate that as particle size decreases, a greater amount of drug is released initially per unit time. After 2 hours, 50 % of the total drug reservoir is released from particles smaller than 40 µm, while particles between 40 and 60 µm release only 40 %. After 8 hours, the smaller (less than 40 µm) particles release essentially 100 % of their drug reservoirs, while the particles of 40 µm-60 µm still have a reservoir of nearly 15 %.

Example 5: Antitussive Preparation
2.10 g of the resinate of Example 1 is mixed with the following:

| | |
|---|---|
| tragacanth | 1.00 g |
| sorbitol | 40.00 g |
| saccharin sodium | 0.10 g |
| 1,2-propylene glycol | 2.50 g |
| methylparaben | 0.12 g |
| propylparaben | 0.03 g |
| sorbic acid | 0.10 g |

water sufficient
to make 100 ml (approx. 64 g)
to arrive at an antitussive formulation having the equivalent of 60.0 mg of dextromethorphan-hydrobromide per 20 ml.

Example 6: Antitussive Tablets

Components per tablet:

| | |
|---|---|
| dextromethorphan-resinate | 187.5 mg |
| lactose D 21 | 40.0 mg |
| polyvinylpolypyrrolidon XL | 70.0 mg |
| Aerosil® | 10.0 mg |
| cellulose HP-M 603 | 40.0 mg |
| Avicel® PH-102 | 40.0 mg |
| magnesiumstearate | 2.5 mg |

The dextromethorphan-resinate, lactose and a part of polyvinyl-pyrrolidon XL as well as Aerosil are mixed and granulated with aqueous cellulose-HPM 603 granulating fluid. A granulate is formed which is pressed into tablets after admixing with Avicel and the rest of the polyvinylpolypyrrolidon XL, Aerosil and magnesium-stearate.

What is claimed is: (for all designated states except Austria, Spain and Greece)

1. A pharmaceutical composition suitable for controlled, sustained release of a pharmaceutically acceptable acid addition salt of dextromethorphan and which pharmaceutical composition contains
a) a cationic, gel-type, microporous ion-exchange resin consisting of a sulfonated polystyrene-divinylbenzene copolymerisate,
b) a pharmaceutically acceptable acid addition salt of dextromethorphan in a weight ratio of 1:5 to 1:10 expressed for the weight ratio of dextromethorphan-hydrobromide and the Na-form of the resin and
c) at least one pharmaceutically acceptable adjuvant.

2. A pharmaceutical composition according to claim 1 wherein the resin is crosslinked with about 4-8 % by weight with divinylbenzene based on the weight of the dry resin.

3. A pharmaceutical composition according to claims 1 or 2 wherein the resin is commercially available and has been approved for pharmaceutical purposes for formulation with medicaments.

4. A pharmaceutical composition according to claim 3, wherein the resin is commercially available under the trade name Amberlite® IRP 69.

5. The composition of claim 1 wherein said average particle size of the resin in dry state is between about 40 μm and about 80 μm.

6. The composition of claim 5 wherein said average particle size is about 50 μm to about 70 μm.

7. The composition of claim 5 or 6 wherein at least 95 % of said resin particles are of said average particle size.

8. A pharmaceutical composition according to claim 1 wherein the dextromethorphan is present in the form of the hydrobromide addition salt.

9. A pharmaceutical composition according to claim 1 wherein dextromethorphan-hydrobromide and the Na-form of the resinate are present in a weight ratio of 1:6.

10. The composition of claim 1 wherein said adjuvants are selected from pharmaceutically acceptable solvents, thickeners, sweeteners, flavorings, colorings, and preservatives.

11. A process for the preparation of pharmaceutical compositions according to claim 1, characterized in that the components a), b) and c) are admixed and formulated into solutions or tablets.

12. Pharmaceutical compositions for use as antitussives in the treatment of humans.

13. Use of a cationic, microporous, ion-exchange resin consisting of a sulfonated polystyrene-divinylbenzene copolymerisate and a pharmaceutically acceptable acid addition salt of dextromethorphan for the preparation of a pharmaceutical composition suitable as antitussively active agent in the treatment of humans.

FO 7.4/RS/sm*

What is claimed is: (for the designated states Austria, Spain and
Greece)

1. A process for the preparation of a pharmaceutical composition
suitable for controlled, sustained release of a pharmaceutically
acceptable acid addition salt of dextromethorphan in solution and
which pharmaceutical composition contains
a) a cationic, gel-type, microporous ion-exchange resin consisting
of a sulfonated polystyrene-divinylbenzene copolymerisate,
b) a pharmaceutically acceptable acid addition salt of dextromethorphan in a weight ratio of 1:5 to 1:10 expressed for the weight
ratio of dextromethorphanhydrobromide and the Na-form of the resin
and
c) at least one pharmaceutically acceptable adjuvant, characterized
in that the components a), b) and c) are admixed and formulated to
administration forms.

2. A process for the preparation of a pharmaceutical composition
according to claim 1 wherein the resin is crosslinked with about
4-8 % by weight with divinylbenzene based on the weight of the dry
resin.

3. A process for the preparation of a pharmaceutical composition
according to claims 1 or 2 wherein the resin is commercially
available and has been approved for pharmaceutical purposes for
formulation with medicaments.

4. A process for the preparation of a pharmaceutical composition
according to claim 3, wherein the resin is commercially available
under the trade name Amberlite® IRP 69.

5. A process for the preparation of a pharmaceutical composition of
claim 1 wherein said average particle size of the resin in dry state
is between about 40 µm and about 80 µm.

6. A process for the preparation of a pharmaceutical composition of claim 5 wherein said average particle size is about 50 µm to about 70 µm.

7. A process for the preparation of a pharmaceutical composition of claim 5 or 6 wherein at least 95 % of said resin particles are of said average particle size.

8. A process for the preparation of a pharmaceutical composition according to claim 1 wherein the dextromethorphan is present in the form of the hydrobromide addition salt.

9. A process for the preparation of a pharmaceutical composition according to claim 1 wherein dextromethorphan-hydrobromide and the Na-form of the resinate are present in a weight ratio of 1:6.

10. A process for the preparation of a pharmaceutical composition of claim 1 wherein said adjuvants are selected from pharmaceutically acceptable solvents, thickeners, sweeteners, flavorings, colorings, and preservatives.

FO 7.4/RS/sm*